(19) Europäisches Patentamt
European Patent Office
Office européen des brevets

(11) Veröffentlichungsnummer: **0 286 969 B1**

(12) **EUROPÄISCHE PATENTSCHRIFT**

(45) Veröffentlichungstag der Patentschrift: **03.06.92**

(51) Int. Cl.$^5$: **C07C 61/40**, C07C 51/00

(21) Anmeldenummer: **88105501.6**

(22) Anmeldetag: **07.04.88**

(54) **Verfahren zur Herstellung von trans-2,2-Dimethyl-3-(2,2-dichlorvinyl)-cyclopropancarbonsäure.**

(30) Priorität: **16.04.87 DE 3712988**

(43) Veröffentlichungstag der Anmeldung:
**19.10.88 Patentblatt 88/42**

(45) Bekanntmachung des Hinweises auf die Patenterteilung:
**03.06.92 Patentblatt 92/23**

(84) Benannte Vertragsstaaten:
**AT BE CH DE FR GB IT LI NL**

(56) Entgegenhaltungen:
**EP-A- 0 095 696**

(73) Patentinhaber: **BAYER AG**
**W-5090 Leverkusen 1 Bayerwerk(DE)**

(72) Erfinder: **Lantzsch, Reinhard, Dr.**
**Am Buschhäuschen 51**
**W-5600 Wuppertal 1(DE)**
Erfinder: **Steinbeck, Karl, Dr.**
**12409 Overbrook Road**
**Leawood 66209 Kansas(US)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 286 969 B1

## Beschreibung

Die vorliegende Erfindung betrifft ein neues Verfahren zur Herstellung von trans-2,2-Dimethyl-3-(2,2-dichlorvinyl)-cyclopropancarbonsäure (= trans-Permethrinsäure). Trans-Permethrinsäure ist ein wertvolles Zwischenprodukt für die Herstellung insektizid wirksamer Pyrethroide.

Es ist bereits bekannt, daß man cis/trans-Gemische von 3-vinyl-substituierten 2,2-Dimethylcyclopropan-1-carbonsäuren erhält, wenn man Polyhalogenalkane an 1-Chlor-3,3-dimethyl-pent-4-en-2-on addiert und das daraus erhältliche Gemisch der Halogenketone weiter mit Basen umsetzt.

Bei diesem Verfahren wird ein erheblicher Anteil an unerwünschten cis-Säuren erhalten (vgl. De-OS 31 00 354).

Es ist außerdem bekannt, daß man trans-3-vinyl-substituierte 2,2-Dimethylcyclopropan-1-carbonsäuren erhält, wenn man 4,4-Dimethyl-6-brom-3-halogen-1-hexen-5-one mit Basen bei erhöhter Temperatur umsetzt. (vgl. DE-OS 32 16 723, DE-OS 32 31 814, EP-OS 0 95 047). Bei diesem Verfahren ist die Reinheit der gebildeten trans-Säuren insbesondere deshalb unbefriedigend, da bei erhöhter Temperatur gearbeitet wird.

Es ist weiter bekannt, daß die Dosierungsrichtung auf das Isomerenverhältnis von 3-vinyl-substituierten 2,2-Dimethyl-cyclopropancarbonsäuren von Einfluß ist. Die Bildung von cis-Säuren läßt sich jedoch auch bei diesem Verfahren nicht vollständig ausschließen.

Es wurde nun gefunden, daß man sehr reine trans-2,2-Dimethyl-3-(2,2-dichlorvinyl)-cyclopropancarbonsäure der Formel (I)

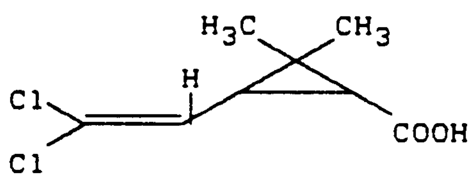

(I)

erhält, indem man Chlorketone der Formel (II)

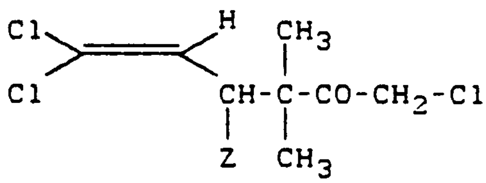

(II)

in welcher

Z für Chlor oder Brom steht,

mit einer wässrigen Lösung von Alkalihydroxid, insbesondere von Natriumhydroxid und/oder Kaliumhydroxid umsetzt.

Die Reaktion ist als überraschend zu bezeichnen, da das aus Tetrachlormethan und 1-Chlor-3,3-dimethyl-pent-4-en-2-on erhaltene Adduktgemisch aus 1,4,6,6,6-Pentachlor-3,3-dimethyl-hexanon-2 und 1,4-Dichlor-4-methyl-3-(2,2,2-trichlorethyl)-pentanon-2 mit Basen überwiegend zu cis-Säuren führt (vgl. DE-OS 31 00 354, Beispiel 4 und 5).

Verwendet man beispielsweise 1,1,3,6-Tetrachlor-4,4-dimethyl-1-hexen-5-on als Ausgangsstoff und wäßrige Natriumhydroxid-Lösung als Base, so kann der Reaktionsverlauf durch folgendes Formelschema wiedergegeben werden:

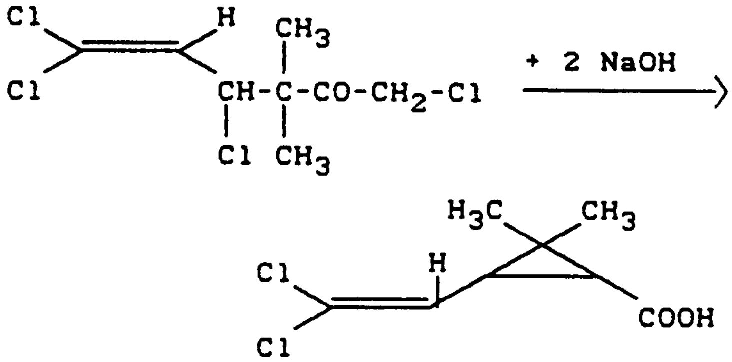


Die bei diesem Verfahren verwendbaren Ausgangsstoffe sind durch die allgemeine Formel (II) definiert. Als besonders bevorzugte Verbindung der Formel II wird

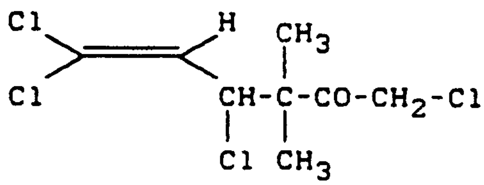

eingesetzt.

Die als Ausgangsstoffe verwendeten Halogenketone der Formel (II) sind teilweise bekannt und/oder lassen sich nach bekannten Verfahren in allgemein üblicher Art und Weise herstellen (vergl. EP-OS 0 95 047). So Läßt sich beispielsweise 1,1,3,6-Tetrachlor-4,4-dimethyl-1-hexan-5-on aus 1,1,3,-Trichlor-4,4-dimethyl-1-hexan-5-on und Chlor herstellen.

Bei der Durchführung des erfindungsgemäßen Verfahrens wird der Ausgangsstoff der Formel (II) in Wasser vorgelegt und die wäßrige Alkalilauge (vorzugsweise Natronlauge oder Kalilauge) zugetropft.

Bei der Durchführung des erfindungsgemäßen Verfahrens setzt man ein Mol Ausgangsstoff der Formel (II) mit 3 bis 10 Mol, vorzugsweise 3 bis 5 Mol Alkalilauge um.

Die Reaktion gelingt überraschenderweise bereits bei Raumtemperatur oder leicht erhöhten Temperaturen.
Im allgemeinen arbeitet man zwischen 10 ° C und 50 ° C, vorzugsweise jedoch zwischen 10 ° C und 30 ° C. Insbesondere wird bei Raumtemperatur umgesetzt.

Die Konzentration der wäßrigen Alkalihydroxidlösung kann zwischen 0,5 % und 20 % liegen, vorzugsweise jedoch zwischen 2,5 % und 10 %. (Bei den Prozentangaben handelt es sich um Gewichtsprozente.)

Die Aufarbeitung erfolgt in üblicher Weise nach allgemein bekannten Methoden, z. B. durch Extraktion des Reaktionsprodukts im organischen, mit Wasser nicht mischbaren Lösungsmittel, Trocknung des Lösungsmittels und Entfernen des Lösungsmittels.

Die trans-Permethrinsäure kann beispielsweise wie folgt zu einem Pyrethroid-Endprodukt mit stark insektizider Wirkung umgesetzt werden:

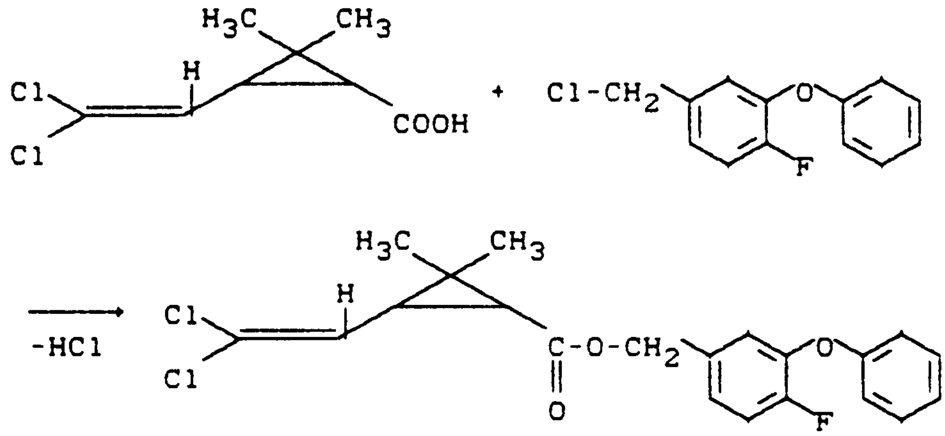


## Herstellungsbeispiele

### Beispiel 1

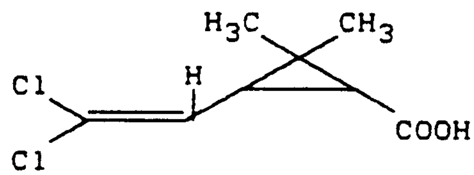

13,2 g 1,1,3,6-Tetrachlor-4,4-dimethyl-1-hexen-5-on (81 %-ig ca. 0,04 Mol) werden in 75 ml Wasser vorgelegt. Dann werden ohne zu kühlen 7,5 g Natriumhydroxid gelöst in 67,5 ml Wasser unter Rühren zugetropft. Dabei steigt die Temperatur von 20 °C auf ca. 26 °C. Man rührt 12 Stunden nach, verdünnt mit Wasser und extrahiert dreimal mit Methylenchlorid. Die Wasserphase wird angesäuert und erneut dreimal mit Methylenchlorid extrahiert. Die vereinigten Extrakte der (sauren) Extraktion werden getrocknet und das Lösungsmittel entfernt. Es bleiben 7,0 g trans-Permethrinsäure (Gehalt: 97 % laut GC, silyliert) zurück. Dies entspricht einer Ausbeute von 81,2 % der Theorie.

### Beispiel 2

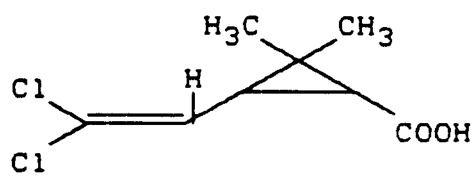

132 g 1,1,3,6-Tetrachlor-4,4-dimethyl-1-hexen-5-on (75 %ig ca. 0,375 Mol) werden in 1050 ml Wasser vorgelegt und bei 25 °C eine Lösung aus 105 g (1,875 Mol) Kaliumhydroxid in 945 ml Wasser zudosiert. Die Reaktion ist nicht exotherm. Man rührt 6 Stunden bei 25 - 27 °C nach, extrahiert dreimal mit Methylenchlorid, säuert die Wasserphase an und extrahiert erneut dreimal mit Methylenchlorid. Die vereinigten Extrakte der sauren Extraktion werden getrocknet und das Lösungsmittel entfernt. Es bleiben 71 g Permethrinsäure zurück (trans-Gehalt laut GC, silyliert: 97 %). Dies entspricht einer Ausbeute von 87,9 % der Theorie).

Herstellung der Ausgangsstoffe:

Beispiel 3

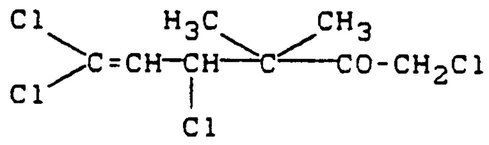

11,5 g (0,05 Mol) 1,1,3-Trichlor-4,4-dimethyl-1-hexen-5-on werden in 50 ml Propionsäure, die etwas Chlorwasserstoff enthält, gelöst. Dann wird bei 0 - 5 °C innerhalb von 15 Minuten 3,6 g (0,05 Mol) Chlor eingeleitet. Man rührt noch 2 Stunden bei 0 - 5 °C nach und destilliert die Propionsäure ab. Der Rückstand wiegt 13,1 g und besteht zu 81 % aus 1,1,3,6-Tetrachlor-4,4-dimethyl-1-hexen-5-on, 8 % Ausgangsstoff und nur 8,5 % Dichlor-keton. Dies entspricht einer Ausbeute von 80,4 % der Theorie.

## Patentansprüche

1. Verfahren zur Herstellung von trans-2,2-Dimethyl-3-(2,2-dichlorvinyl)-cyclopropancarbonsäure der Formel (I)

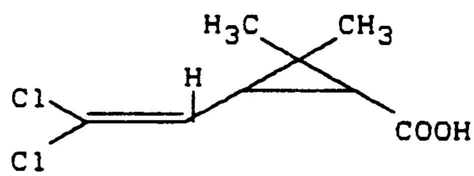

(I)

dadurch gekennzeichnet, daß man Chlorketone der Formel (II)

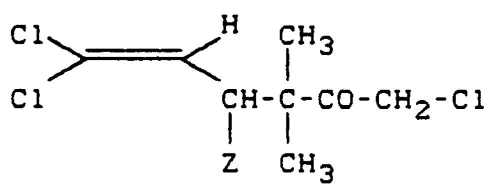

(II)

in welcher

Z für Chlor oder Brom steht,

mit einer wässrigen Lösung von Alkalihydroxid umsetzt.

2. Verfahren gemäß Anspruch 1, dadurch gekennzeichnet, daß man pro Mol Chlorketon der Formel (II) 3 bis 10 Mol Alkalihydroxid einsetzt.

3. Verfahren gemäß Anspruch 1 und 2, dadurch gekennzeichnet, daß man pro Mol Chlorketon der Formel (II) 3 bis 5 Mol NaOH oder KOH einsetzt.

4. Verfahren gemäß Anspruch 1 bis 3, dadurch gekennzeichnet, daß man bei Temperaturen zwischen 10 und 50 °C arbeitet.

5. Verfahren gemäß Anspruch 1 bis 4, dadurch gekennzeichnet, daß die eingesetzte wäßrige Natrium-

bzw. Kaliumhydroxidlösung in einer Konzentration zwischen 0,5 und 20 Gewichtsprozent vorliegt.

**Claims**

1. Process for the preparation of trans-2,2-dimethyl-3-(2,2-dichlorovinyl)-cyclopropanecarboxylic acid of the formula (I)

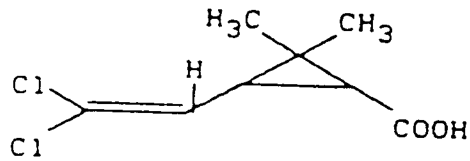

(I)

characterised in that chloroketones of the formula (II)

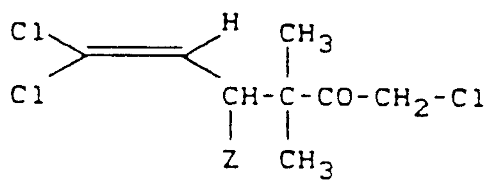

(II)

in which

Z represents chlorine or bromine,

are reacted with an aqueous solution of alkali metal hydroxide.

2. Process according to Claim 1, characterised in that 3 to 10 mol of alkali metal hydroxide are employed per mole of chloroketone of the formula (II).

3. Process according to Claim 1 and 2, characterised in that 3 to 5 mol of NaOH or KOH are employed per mole of chloroketone of the formula (II).

4. Process according to Claim 1 to 3, characterised in that it is carried out at temperatures between 10 and 50 ° C.

5. Process according to Claim 1 to 4, characterised in that the aqueous sodium hydroxide solution or potassium hydroxide solution employed is present in a concentration of between 0.5 and 20 per cent by weight.

**Revendications**

1. Procédé pour la préparation de l'acide trans-2,2-diméthyl-3-(2,2-dichlorovinyl)cyclopropanecarboxylique de formule (I)

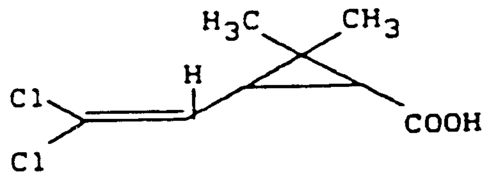

(I)

**caractérisé en ce qu'**on fait réagir des chlorocétones de formule (II)

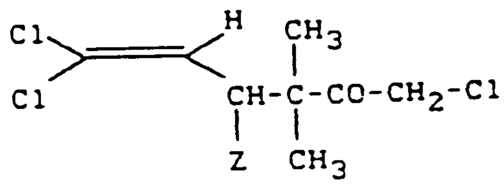

(II)

dans laquelle

Z représente un atome de chlore ou un atome de brome

avec une solution aqueuse d'un hydroxyde de métal alcalin.

2. Procédé selon la revendication 1, **caractérisé en ce que,** par mole de chlorocétone de formule (II), on met en oeuvre de 3 à 10 moles d'hydroxyde de métal alcalin.

3. Procédé selon les revendications 1 et 2, **caractérisé en ce que,** par mole de chlorocétone de formule (II), on met en oeuvre de 3 à 5 moles de NaOH ou de KOH.

4. Procédé selon les revendications 1 à 3, **caractérisé en ce qu'**on travaille à des températures entre 10 et 50 ° C.

5. Procédé selon les revendications 1 à 4, **caractérisé en ce que** la solution aqueuse d'hydroxyde de sodium ou de potassium mise en oeuvre est présente en une concentration entre 0,5 et 20 pour cent en poids.